# EUROPEAN PATENT APPLICATION

(11) **EP 4 442 777 A1**
(43) Date of publication of application: **09.10.2024**
(21) Application number: 23166514.2
(22) Date of filing: 04.04.2023
(51) Int. Cl.: C09D 11/52, C09D 11/10

(54) **ELECTRICALLY CONDUCTIVE INK COMPOSITION**

(71) Applicant: Henkel AG & Co. KGaA, 40589 Düsseldorf (DE)
(72) Inventor: Goethel, Frank, 09125 Chemnitz (DE); Negele, Carla, 41472 Neuss (DE); Oldenzijl, Rudolf, 9601 Hoogezand (NL); Matthijssen, Tjalle, 9736TN Groningen (NL)

(57) **Abstract**

The present invention relates to an electrically conductive ink composition comprising a) a resin; b) a solvent; and c) AgCl coated silver particles. The conductive ink composition according to the present invention provides good adhesion to polymer substrates, a good electrical conductivity, as well as a good compatibility and stability with high salt content hydrogels. The electrically conductive ink composition according to the present invention is suitable for use in medical applications such as hydrogel containing biosignal sensing electrodes and as reference/counter electrodes for electrochemical sensors.

## Description

### Technical field

The present invention relates to an electrically conductive ink composition comprising AgCl coated silver particles.

### Technical background

Ordinary commercially available electrically conductive printable inks containing a blend of electrically conductive silver (Ag) particles and non-conductive silver chloride (AgCI) particles have been on a market for many years. These printable inks have been commonly used in commercially available medical electrodes.

The electrically conductive printable inks function in medical electrodes as reference/counter electrodes for electrochemical sensors such as glucose sensors, pH sensors, ion selective electrode systems, and in hydrogel-containing sensing applications such as biosignal sensing electrodes. Biosignals are produced by the electrical activity that arises from the biological activity that takes place within different tissues and organs of the human body. Most common types of biosignal sensing methods are ECG (electrocardiography), EEG (electroencephalography) and EMG (electromyography). The electrically conductive printing inks are used to form a conductive layer on a substrate of choice after printing and thermal treatment. The electrically conductive printable inks with a high AgCl quantity from 30 to 40% are preferred in biosensor applications, because they provide sufficient AgCI quantity for the Ag/AgCI redox reaction. However, in biosignal sensing electrode applications, such printable inks with a high AgCl quantity are not usually used because of the weak stability towards used hydrogels covering the Ag/AgCI layer. The hydrogels contain salts such as potassium chloride, sodium chloride, magnesium sulphate or magnesium acetate and are used as skin contact layer to transfer the biomedical signal from the human body to the device. However, the Ag/AgCI layer which is in contact with the hydrogel loses its electrical conductivity over time, most likely because of oxidation processes of the silver particles. Ordinary hydrogels contain a high concentration of salts and often have a low pH leading to an accelerated oxidation of Ag.

Printable Ag/AgCI inks with low AgCl quantity from 10 to 20 % are typically used in the current applications containing a high salt content hydrogel. Even with a relatively low AgCl content these materials suffer from degradation when in contact with hydrogel. To remain the electrical conductivity of the Ag/AgCI layer in contact with hydrogel, the Ag/AgCI layer is placed often on top of a non-silver based electrically conductive layer containing carbon black and graphite.

Ag/AgCI inks based on thermosetting (epoxy-based) resin systems are available from other suppliers claiming that the material is more chemically resistant and showing better stability with hydrogel. Those inks, however, usually require high curing temperatures greater than 120°C for longer than 15 minutes limiting the choice of substrate having a negative impact on energy consumption, manufacturing speed and wear comfort as common flexible substrates based on thermoplastic polyurethanes are not temperature-resistant enough. In addition, thermosetting inks usually have limited shelf life especially if they are designed to be cured at relatively low temperatures less than 150°C and requires storage below room temperature. Thermoset inks are often based on monomers which are not biocompatible. They contain stabilizers, curing agents or catalysts that are not considered to be skin friendly.

Therefore, there is a need for an electrically conductive printable ink which provides an electrically conductive Ag/AgCI layer with a high AgCl surface availability, while providing a good compatibility and stability in combination with an high salt containing hydrogel required in biosignal sensing electrodes.

### Short description of the figures

Figure 1 illustrates the performance of the examples (Ag/AgCI layer) in combination with a solid sensing hydrogel (Impedance Z).
Figure 2 illustrates the performance of the examples (Ag/AgCI layer) in combination with a solid sensing hydrogel (Impedance phase angle).
Figure 3 illustrates the availability of AgCl on the surface of the layer.

### Summary of the invention

The present invention relates to an electrically conductive ink composition comprising a) a resin; b) a solvent; and c) AgCl coated silver particles.

The present invention relates to a dried electrically conductive ink composition according to the present invention.

The present invention also encompasses use of the electrically conductive ink composition or dried electrically conductive ink composition according to the present invention in hydrogel containing biosignal sensing electrodes and as reference/counter electrodes for electrochemical sensor.

### Detailed description of the invention

In the following passages the present invention is described in more detail. Each aspect so described may be combined with any other aspect or aspects unless clearly indicated to the contrary. In particular, any feature indicated as being preferred or advantageous may be combined with any other feature or features indicated as being preferred or advantageous.

In the context of the present invention, the terms used are to be construed in accordance with the following definitions, unless a context dictates otherwise.

As used herein, the singular forms "a", "an" and "the" include both singular and plural referents unless the context clearly dictates otherwise.

The terms "comprising", "comprises" and "comprised of' as used herein are synonymous with "including", "includes" or "containing", "contains", and are inclusive or open-ended and do not exclude additional, non-recited members, elements or method steps.

As used herein, the term "*consisting of*" excludes any element, ingredient, member or method step not specified.

The words "*preferred*", "*preferably*", "*desirably*" and "*particularly*" are used frequently herein to refer to embodiments of the disclosure that may afford particular benefits, under certain circumstances. However, the recitation of one or more preferable, preferred, desirable or particular embodiments does not imply that other embodiments are not useful and is not intended to exclude those other embodiments from the scope of the disclosure.

As used throughout this application, the word "*may*" is used in a permissive sense - that is meaning to have the potential to - rather than in the mandatory sense.

The recitation of numerical end points includes all numbers and fractions subsumed within the respective ranges, as well as the recited end points.

All percentages, parts, proportions and then like mentioned herein are based on weight unless otherwise indicated.

When an amount, a concentration or other values or parameters is/are expressed in form of a range, a preferable range, or a preferable upper limit value and a preferable lower limit value, it should be understood as that any ranges obtained by combining any upper limit or preferable value with any lower limit or preferable value are specifically disclosed, without considering whether the obtained ranges are clearly mentioned in the context.

All references cited in the present specification are hereby incorporated by reference in their entirety.

Unless otherwise defined, all terms used in disclosing the invention, including technical and scientific terms, have the meaning as commonly understood by one of the ordinary skilled in the art to which this invention belongs to. By means of further guidance, term definitions are included to better appreciate the teaching of the present invention.

The present invention relates to an electrically conductive ink composition comprising a) a resin; b) a solvent; and c) AgCl coated silver particles.

The Applicant has found that the use of silver chloride coated silver particles as an electrically conductive component provides several advantages compared to an ordinarily used blend of silver particles and silver chloride particles. The silver chloride coated silver particles disperse easier into composition compared to silver chloride particles. Further, the printable ink composition comprising silver chloride coated silver particles has lower viscosity and thixotropy, which enables to increase the silver particle quantity in the composition. The overall quantity of the electrically conductive particles in the ink composition is higher when silver chloride coated silver particles are used alone, compared to a mixture of electrically conductive silver particles and non-conductive silver chloride particles. This provides a better compatibility and stability with ionically conductive hydrogels. Further, it was found that a higher AgCl quantity in the ink composition according to the present invention led to a higher AgCl availability at the surface of the printed layer, decreased the interface resistance between the electrically conductive Ag/AgCI layer and ionically conductive hydrogel. In addition, a lower impedance at frequencies lower than 10 Hz, was achieved, providing a better biosignal quality and a faster voltage discharge after defibrillation required for ECG electrodes ("Defibrillation overload recovery test" according to ANSI/AAMI EC12:2000).

The electrically conductive ink composition according to the present invention comprises a resin. The resin adheres the ink to the substrate.

Suitable resin for use in the present invention may be selected from the group consisting of thermoplastic polyurethanes, polyesters, polyacrylates, polysiloxanes, halogenated vinyl or vinylidene polymers, polyamide copolymers, phenoxy resins, polyethers, polyketones, polyvinyl butyral, polyvinyl pyrrolidone, polycyanoacrylates and mixtures thereof, preferably, said binder selected from the group consisting of thermoplastic polyurethanes, phenoxy resins and mixtures thereof.

Thermoplastic polyurethanes are preferred because they are flexible, and therefore, ideally suitable for wearables as they do not tend to crack upon bending or stretching. Thermoplastic polyurethanes are also compatible with TPU and PET substrates. Sometimes inks based on thermoplastic polyurethanes may be a bit on softer side and may not provide an ideal mechanical stability. To improve the mechanical stability, a combination of thermoplastic polyurethanes and phenoxy resins may be used as the combination provides a mechanically stable but flexible ink.

In a highly preferred embodiment, the resin is a combination of thermoplastic polyurethane and phenoxy resin.

Suitable commercially available resins for use in the present invention include but are not limited to ESTANE 5715 and ESTANE 5703 from Lubrizol Company and PKHB, PKHJ and PKHC from Huntsman Advanced Materials.

The resin may be present in an electrically conductive ink composition according to the present invention in a quantity from 0.1 to 15 wt.% by weight of the total weight of the composition, preferably from 2 to 10 wt.% and more preferably from 4 to 9 wt.%.

The above defined quantities are preferred, because too low quantity of the resin, less than 0.1%, may lead to poor adhesion of the ink composition to the substrate, whereas too high quantity, more than 15% may lead to decreased electrical conductivity, compatibility with hydrogel and AgCl availability.

The electrically conductive ink composition according to the present invention comprises a solvent.

Suitable solvent may be selected based on the printing method to be used to apply the printable ink onto a surface. For example, solvents with higher boiling point are generally used in screen printing to prevent drying of the ink on the screen. And solvents with lower boiling point are generally used in high-speed printing, e.g. flexographic and rotogravure printing because the drying speed of the ink is limiting the printing speed.

Suitable solvent for use in the present invention may be selected from the group consisting of 2-butoxyethyl acetate, 2-(2-butoxyethoxy)ethanol, [2-(2-butoxyethoxy)-ethyl]acetate, 2-butoxyethanol, isophorone, 3,3,5-trimethyl-2-cyclohexene-1-one, dimethyl succinate, dimethyl glutarate, dimethyl adipate, acetic acid, 1-methoxypropan-2-ol, 1-methoxy-2-propylacetat, dipropylene glycol (mono)methyl ether, toluene, ethanol, isopropyl alcohol, n-propanol, ethoxy propanol, ethyl acetate, butyl acetate, n-propyl acetate, isopropyl acetate, methyl ethyl ketone, methyl isobutyl ketone, water, glycol ethers and mixtures thereof.

Particularly suitable solvents for screen printing are selected from the group consisting of 2-butoxyethyl acetate, 2-butoxyethanol, 2-(2-butoxyethoxy)ethanol, [2-(2-butoxyethoxy)-ethyl]acetate, dimethyl succinate, dimethyl glutarate, dimethyl adipate, acetic acid, dipropylene glycol (mono)methyl ether, glycol ethers and mixtures thereof, preferably selected from 2-butoxyethyl acetate, 2-butoxyethanol, dibasic esters and mixtures thereof.

Above mentioned solvents are preferred because they dissolve the resin well, are not harmful and allow sufficient opening times on the screen.

Particularly suitable solvents for high-speed printing are selected from the group consisting of ethanol, isopropyl alcohol, n-propanol, ethoxy propanol, ethyl acetate, butyl acetate, n-propyl acetate, isopropyl acetate, 1-methoxypropan-2-ol, 1-methoxy-2-propylacetat, methyl ethyl ketone, methyl isobutyl ketone, water and mixtures thereof, preferably selected from n-propyl acetate, butyl acetate, 1-methoxypropan-2-ol, 1-methoxy-2-propylacetat and mixtures thereof.

Above mentioned solvents are preferred because they dissolve the resin well and are easily released during the drying process.

Suitable commercially available solvents for use in the present invention include but are not limited to 2-butoxyethyl acetate (=EB acetate) and 1-methoxy-2-propylacetat (=PM acetate) from Eastman Chemical Company.

The solvent may be present in an electrically conductive ink composition in a quantity from 5 to 80 wt.% by weight of the total weight of the composition, preferably from 10 to 60 wt.% and more preferably from 20 to 50 wt.%.

These solvent quantities are preferred because if the solvent quantity is below 5% the viscosity of the printable ink composition may be too high and the ink is not suitable for printing, whereas, if the solvent quantity is more than 80% the viscosity may be too low, and the dried ink layer may be too thin.

The electrically conductive ink composition according to the present invention comprises AgCl coated silver particles.

In a highly preferred embodiment, the electrically conductive ink composition according to the present invention is free of silver particles, meaning that the sole source of electrically conductive particles is silver chloride coated silver particles.

The AgCl content in the AgCl coated silver particles may be from 1 to 80 % by weight based on the total weight of the silver particles, preferably from 2 to 75% and from 3 to 70 more preferably from 5 to 50 %.

The above AgCl quantities are preferred because too low quantity, less than 1%, may lead to too high impedance at low frequencies of biosignal sensing electrodes such as disposable ECG electrodes. This is due a higher interface resistance between the hydrogel and Ag/AgCI dry layer, which mainly impacts the defibrillation overload recovery performance and the ECG signal on short time intervals. Whereas too high quantity, more than 80%, may lead to too high electrical resistance of the Ag/AgCI, which may make the electrode unsuitable for medical applications.

The shape of the particle is not limited to any particular shape, and any shape of the AgCl coated silver particles may be used. The silver chloride coated silver particles may have a flake shape, for example the flakes can be in the geometric form of flake, dendritic, or needle type filler flakes. The AgCl coated silver particles can also be spherical, cubical or an irregular shape. The AgCl coated silver particles can also be a combination of conductive fillers with different shape, for example a combination of flake and spherical particles. Preferably the AgCl coated silver particles are flake shape.

Flake shape is preferred because flake shape particles form stacks in the layers leading to improved flexibility and better conductivity at lower concentrations.

The silver chloride coated silver particles preferably have an average particle size of from 1 µm to 20 µm, wherein particle size D₅₀ value is measured by laser diffractometry.

As used herein, the term "average particle size" refers to the D₅₀ value of the cumulative volume distribution curve at which 50% by volume of the particles have a diameter less than said value. The average particle size is measured in the present invention through laser diffractometry preferably using a Malvern Mastersizer 3000 available from Malvern Instruments Ltd. or Horiba LA-950V2 from Horiba Ltd.. In this technique, the size of particles in suspensions or emulsions is measured using the diffraction of a laser beam, based on application of either Fraunhofer or Mie theory. In the present invention, Mie theory or a modified Mie theory for non-spherical particles is applied and the particle sizes relate to scattering measurements at an angle from 0.02 to 135 degrees relative to the incident laser beam.

Particle size of from 1 µm to 20 µm is preferred because with the smaller particle size the thickness of the AgCl coating may be either too thin or too thick and the amount of silver in the core may not be enough for sufficient conductivity. Whereas too large particle size may lead to increased surface roughness and decreased printing quality and larger particles may also block the printing screen.

The AgCl coated silver particles may have a tap density of from 2.0 g/cm³ to 6.0 g/cm³, wherein the tap density is determined in accordance with ISO 3953, using a 25 cm³ graduated glass cylinder.

The AgCl coated silver particles may have a specific surface area of from 0.1 m²/g to 1.6 m²/g, wherein the specific surface area is measured according to ASTM B922-10 widely using nitrogen as an analysis gas. The term "specific surface area" as used herein refers to the BET specific surface area which is determined in accordance with ASTM B922-10 widely using nitrogen as an analysis gas.

Suitable commercially available silver chloride coated silver particles for use in the present invention include but are not limited to SFACGL-09, SFAGCL-17 and SFAGCL-69 from Ames Goldsmith Advanced Materials Corporation.

The AgCl coated silver particles may be present in an electrically conductive ink composition according to the present invention in an amount from 20 to 90 wt.% by weight based on the total weight of the composition, preferably from 30 to 85 wt.%, more preferably from 45 to 80 wt.% and even more preferably from 50% to 75%.

These AgCl coated silver particles quantities are preferred because if the particle quantity is below 20%, this may lead to too low viscosity and thin printed layers or a low filler/resin ratio leading to low conductivity. Whereas quantities, more than 90%, may lead to too high viscosity leading to insufficient printability, poor adhesion properties and low flexibility.

In a preferred embodiment the filler/resin ratio is greater than 7, preferably greater than 10, and even more preferably greater than 12.

The Applicant has found that relatively high filler/resin ratio is key reason for the good performance of the electrically conductive ink composition according to the present invention.

The electrically conductive ink composition according to the present invention may further comprise additives that can impart improved properties to this composition. For instance, the additives may impart one or more of: improved elastic properties; improved elastic recovery; improved adhesion; longer enabled processing time; faster curing time; film forming properties, and lower residual tack.

The electrically conductive ink composition according to the present invention may further comprise one or more component selected from the group consisting of surface-active agents, surfactants, wetting agents, antioxidants, thixotropes, reinforcement fibres, silane functional perfluoroethers, phosphate functional perfluoroethers, titanates, waxes, phenol formaldehyde, air release agents, flow additives, adhesion promoters, rheology modifiers, and spacer beads and mixtures thereof. The additional ingredients are optional and are specifically chosen to obtain any desired properties for the chosen end use. When used, the additives may comprise up to 10 weight percent of the total dry composition.

The viscosity of the electrically conductive ink composition according to the present invention may have a viscosity from 0.5 to 30 Pa.s, wherein the viscosity is measured according with ISO 3219 using a rheometer at a constant shear rate of 15-s with a 20 mm plate-plate configuration (0.2 mm gap, 60 sec, at 25 °C).

Particularly suitable viscosity for screen- and roto screen printing is preferably within the range of from 2 to 30 Pa s.

Particularly suitable viscosity for rotogravure or flexographic printing, a suitable viscosity is within the range of from 0.5 to 4 Pa s.

The Applicant has found that the above defined viscosity range is ideal for all printing methods and the ink composition is printable. Too low or too high viscosities have generally negative impact on printability and printing quality.

The electrically conductive ink composition according to the present invention may be printed on to a surface by using a method selected from the group consisting of screen printing, roto screen printing, rotogravure printing and flexographic printing.

The present invention also relates to a dried electrically conductive ink composition according to the present invention.

Once the electrically conductive ink composition according to the present invention is printed on to a surface of a substrate, the ink is dried. The drying is carried out at a temperature no greater than about 120° C for a time in the range of from six minutes up to about 2 hours.

The electrically conductive ink composition or dried electrically conductive ink composition according to the present invention may be used in hydrogel containing biosignal sensing electrodes and as reference/counter electrodes for electrochemical sensor.

Examples of such electrochemical electrodes and sensors are glucose sensors, pH sensors, ion selective electrode systems such as fluoride selective electrodes.

Examples of such hydrogel containing biosignal sensing electrodes using methods such as ECG (electrocardiography), EEG (electroencephalography) and EMG (electromyography). Other examples are defibrillation electrodes.

### Examples

The examples described hereunder exemplify the properties of the compositions according to the embodiments of the present invention. Unless otherwise indicated, all parts and percentages in following examples, as well as throughout the description, are parts by weight or percentages by weight respectively. The following examples are included for purposes of illustration so that the disclosure may be more readily understood and are in no way to be intended to limit the scope of the disclosure unless otherwise specifically indicated.

### Example 1

All samples were prepared according to the formula details presented in table 1 below. Compositions were prepared by following process: the thermoplastic resins were dissolved in the solvent using a mechanical stirrer, followed by the addition of the fillers. Subsequently compositions were three roll milled using a final gap of 10 µm before printing them on PET substrate (AUTOSTAT CUS5) using a screen with a mesh count of 100 per cm (250 per inch). The prints were dried at 120°C for 15 minutes.

**Table 1**

| | Comparative example 1 | Comparative example 2 | Example 1 | Example 2 |
|---|---|---|---|---|
| | Ag/AgCl ratio 9:1; P/B ratio 8 | Ag/AgCl ratio 6:4; P/B ratio 8 | Ag/AgCl ratio 9:1; P/B ratio 8 | Ag/AgCl ratio 7:3; P/B ratio 12 |
| Phenoxy resin (PKHJ, from Huntsman Advanced Materials) | 5.06 | 5.06 | 5.06 | 3.84 |
| Polyurethane aromatic resin (Estane 5715, from Lubrizol) | 2.53 | 2.53 | 2.53 | 1.92 |
| 2-Butoxyethyl acetate (from VWR International, LLC) | 33.05 | 33.05 | 33.05 | 25.08 |
| Ag flakes (SF 7E, from Ames Goldsmith) | 53.42 | 35.62 | | |
| AgCl powder (SEAG 123, from Metalor) | 5.94 | 23.74 | | |
| AgCl coated Ag flakes (SFAGCL-09, from Ames Goldsmith) | | | 55.20 | 33.89 |
| AgCl coated, Ag flakes (SFAGCL-50 from Ames Goldsmith) | | | 1.78 | 35.26 |

### Example 2 Impedance spectra

Hydrogel "AG635" from Axelgaard Manufacturing Co., Ltd. with a dimension of 2 cm x 2 cm was applied on to the surface of the conductive layer (3 cm x 2 cm) prepared from the examples represented in table 1. The final sample built up for the measurement was Ag/AgCI layer + Hydrogel + Ag/AgCI layer. Impedance was measured with potentiostat from Metrohm Autolab at a frequency range from 100000 to 0.1 Hz.

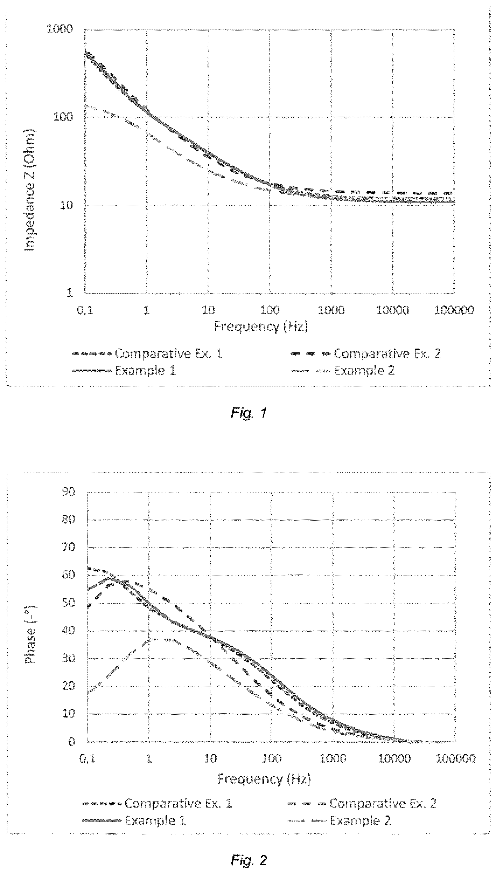

Figure 1 (Impedance Z) and Figure 2 (Impedance phase angle) illustrate the performance of the examples (Ag/AgCI layer) in combination with a solid sensing hydrogel (AG-635, Axelgaard Manufacturing).

The results showed that the impedance spectra were similar for the comparative example 1 and example 1, indicating that AgCl coated silver flakes can perform comparatively to ordinary silver flake and AgCl powder mixture. Example 2 shows the advantage of a high load composition, as it lowers the interface resistance (lower phase angle shown in Fig 2) between the conductive Ag/AgCI layer and the hydrogel. This leads to lower impedances at frequencies below 100 Hz, which desired for better ECG signal quality and faster discharge after defibrillation.

### Example 3 Stability with hydrogel at high elevated temperature

The compositions from example 1 were stored into a box oven at 55°C. The impedance of the samples was measured after 1 day, 7 days and 21 days. Table 2 reports the impedance at 10 Hz of the examples (average of 3 samples).

**Table 2**

| | Comparative example 1 | Comparative example 2 | Example 1 | Example 2 |
|---|---|---|---|---|
| Initial | 42 | 41 | 48 | 27 |
| After 1 day @ 55°C | 36 | 37 | 42 | 34 |
| After 7 days @ 55°C | 376 | 308260 | 382 | 414 |
| After 21 days @ 55°C | 1429 | 357492 | 1570 | 1540 |

The comparative examples exemplify that increasing AgCl quantity leads to a very poor hydrogel compatibility. Example 2 according to the present invention exemplifies that with AgCl coated silver also higher AgCl amounts are possible as the aging in hydrogel is very similar to Comparative Example 1 with a Ag/AgCI ratio of 9:1. The initial impedance at 10 Hz is lower than in comparative Example 1 showing the benefit of a higher AgCl quantity.

### Example 4 AgCl depletion time / determine AgCl surface availability

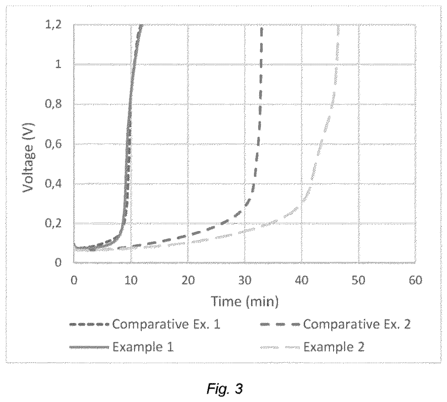

The hydrogel (AG-635, from Axelgaard) with a dimension of 2 cm x 2 cm was applied to the surface of the Ag/AgCI layer (3 cm x 2 cm) made of the compositions from example 1. Another conductive layer on PET (Autostat CUS5) was made of Ag using LOCTITE EDAG PF 410 from Henkel AG & Co. KGaA and was attached on the other side of the hydrogel. The Ag/AgCI layer acted as anode, the Ag layer acted as cathode. The final sample built up for the measurement was Ag/AgCI layer + Hydrogel + Ag layer (made with LOCTITE EDAG PF 410). A constant current of 1mA was applied to the sample, leading to a transfer of Cl anions to the Anode made of Ag only. Figure 3 illustrates the availability of AgCl on the surface of the layer. An increase in voltage was observed once all chloride from the Ag/AgCI layer has been depleted.

## Claims

1. An electrically conductive ink composition comprising
a) a resin;
b) a solvent; and
c) AgCl coated silver particles.

2. The electrically conductive ink composition according to claim 1, wherein the composition is free of silver particles.

3. The electrically conductive ink composition according to claim 1 or 2, wherein the resin is selected from the group consisting of thermoplastic polyurethanes, polyesters, polyacrylates, polysiloxanes, halogenated vinyl polymers, halogenated vinylidene polymers, polyamide copolymers, phenoxy resins, polyethers, polyketones, polyvinyl butyral, polyvinyl pyrrolidone, polycyanoacrylates and mixtures thereof, preferably, selected from the group consisting of thermoplastic polyurethanes, phenoxy resins and mixtures thereof

4. The electrically conductive ink composition according to any one of claims 1 to 3, wherein the resin is present from 0.1 to 15 wt.% by weight based on the total weight of the composition, preferably from 2 to 10 wt.% and more preferably from 4 to 9 wt.%.

5. The electrically conductive ink composition according to any one of claims 1 to 4, wherein the solvent is selected from the group consisting of 2-butoxyethyl acetate, 2-(2-butoxyethoxy)ethanol, [2-(2-butoxyethoxy)-ethyl]acetate, 2-butoxyethanol, isophorone, 3,3,5 trimethyl-2-cyclohexene-1-one, dimethyl succinate, dimethyl glutarate, dimethyl adipate, acetic acid, 1-methoxypropan-2-ol, 1-methoxy-2-propylacetat, dipropylene glycol (mono)methyl ether, toluene, ethanol, isopropyl alcohol, n-propanol, ethoxy propanol, ethyl acetate, butyl acetate, n-propyl acetate, isopropyl acetate, methyl ethyl ketone, methyl isobutyl ketone, water, glycol ethers and mixtures thereof.

6. The electrically conductive ink composition according to any one of claims 1 to 5, wherein the solvent is present from 5 to 80 wt.% by weight based on the total weight of the composition, preferably from 10 to 60 wt.% and more preferably from 20 to 50 wt.%.

7. The electrically conductive ink composition according to any one of claims 1 to 6, wherein the AgCl content in the AgCl coated silver particles is from 1 to 80 % by weight based on the total weight of the particles, preferably from 2 to 75 %, more preferably from 3 to 70% and even more preferably from 5 to 50%.

8. The electrically conductive ink composition according to any one of claims 1 to 7, wherein the AgCl coated silver particles have a particle size of from 1 µm to 20 µm, wherein particle size D₅₀ value is measured by laser diffractometry.

9. The electrically conductive ink composition according to any one of claims 1 to 8, wherein the AgCl coated silver particles have a tap density of from 2.0 g/cm³ to 6.0 g/cm³, wherein the tap density is determined in accordance with ISO 3953, using a 25 cm³ graduated glass cylinder.

10. The electrically conductive ink composition according to any one of claims 1 to 9, wherein the AgCl coated silver particles have a specific surface area of from 0.1 m²/g to 1.6 m²/g, wherein the specific surface area is measured according to ASTM B922-10 widely using nitrogen as an analysis gas.

11. The electrically conductive ink composition according to any one of claims 1 to 10, wherein the AgCl coated silver particles are present from 20 to 90 wt.% by weight based on the total weight of the composition, preferably from 30 to 85 wt.%, preferably from 35 to 80 wt.%, more preferably from 40 to 75 wt.% and even more preferably from 50% to 75%.

12. The electrically conductive ink composition according to any one of claims 1 to 11, wherein the ink may further comprise one or more component selected from the group consisting of surface-active agents, surfactants, wetting agents, antioxidants, thixotropes, reinforcement fibres, silane functional perfluoroethers, phosphate functional perfluoroethers, titanates, waxes, phenol formaldehyde, air release agents, flow additives, adhesion promoters, rheology modifiers, and spacer beads.

13. The electrically conductive ink composition according to any one of claims 1 to 12, wherein the ink has a viscosity of from 0.5 to 30 Pa.s, wherein the viscosity is measured according to ISO 3219 using a rheometer at a constant shear rate of 15-s with a 20 mm plate-plate configuration (0.2 mm gap, 60 sec, at 25 °C).

14. A dried electrically conductive ink composition according to any one of claims 1 to 13.

15. Use of the electrically conductive ink composition according to any one of claims 1 to 13 or dried electrically conductive ink composition according claim 14 in in hydrogel containing biosignal sensing electrodes and as reference/counter electrodes for electrochemical sensors.
